Europäisches Patentamt

**European Patent Office**

Office européen des brevets

(11) Publication number: **0 036 351**
**B1**

# EUROPEAN PATENT SPECIFICATION

(45) Date of publication of patent specification: **02.01.85**

(51) Int. Cl.⁴: **A 61 K 9/06**

(21) Application number: **81400326.5**

(22) Date of filing: **03.03.81**

(54) **Ophthalmic compositions of carbonic anhydrase inhibitors for topical application in the treatment of elevated intraocular pressure.**

(30) Priority: **04.03.80 US 128439**

(43) Date of publication of application:
**23.09.81 Bulletin 81/38**

(45) Publication of the grant of the patent:
**02.01.85 Bulletin 85/01**

(84) Designated Contracting States:
**AT BE CH DE FR GB IT LI LU NL SE**

(56) References cited:
**EP-A-0 014 642**
**EP-A-0 033 042**
**FR-A-2 281 757**
**FR-A-2 332 008**
**GB-A- 851 287**
**GB-A- 860 371**
**US-A-4 008 719**

**CHEMICAL ABSTRACTS, vol. 89, no. 5, July 31, 1978, page 46, abstract 36692d Columbus, Ohio, USA R. KIMURA: "Effect of long-term subconjunctival administration of Diamox (acetazolamide) on the ocular tension in rabbit"**

(73) Proprietor: **MERCK & CO. INC.**
**126, East Lincoln Avenue P.O. Box 2000**
**Rahway New Jersey 07065 (US)**

(72) Inventor: **Smith, Robert L.**
**1355 Pickwick Lane**
**Lansdale Pennsylvania 19446 (US)**

(74) Representative: **Corre, Jacques Denis Paul et al**
**Cabinet Regimbeau 26, Avenue Kléber**
**F-75116 Paris (FR)**

Courier Press, Leamington Spa, England.

## Description

Disclosure of the invention

This invention relates to compositions of carbonic anhydrase inhibitors that are topically effective in the treatment of elevated intraocular pressure. More particularly, it relates to an ophthalmologically acceptable water soluble polymeric insert for lowering intraocular pressure, characterized in that it comprises a mixture of a water soluble ophthalmologically acceptable non-toxic polymer and an intraocular pressure lowering amount of a mono alkali metal salt of a dibasic carbonic anhydrase inhibitor selected from

4,5-dichloro-m-benzene-disulfonamide;
N-[5-(aminosulfonyl)-3-methyl-1,3,4-thiadiazol-2(3H)-ylidene]acetamide;
p-sulfamoylbenzoic acid;
N-[5-(aminosulfonyl)1,3,4-thiadiazol-2-yl]propanamide;
N-[5-(aminosulfonyl)-1,3,4-thiadiazol-2-yl]butanamide and
5-benzenesulfonamido-1,3,4-thiadiazol-2-sulfonamide.

This ophthalmologically water soluble insert is especially useful in the treatment of hypertension and glaucoma.

The state of the art can be illustrated by FR—A—2 332 008 and Chemical Abstracts 89 (1978): 36691c.

Glaucoma is a degenerative disease of the eye wherein the pressure within the eye (i.e., intraocular pressure) is too high for the normal function of the eye and, as a result, damage occurs to the optic nerve head resulting in irreversible loss of visual function. If untreated, glaucoma will eventually lead to blindness. Ocular hypertension, i.e., the condition of elevated intraocular pressure without optic nerve head damage or characteristic glaucomatous visual field loss, is now believed by the majority of ophthalmologists to merely represent the earliest phase in the onset of a glaucoma.

A number of the drugs presently employed to treat glaucoma are not entirely satisfactory, particularly in the earliest course of the disease when the side effects they produce are often worse than the symptoms of the disease.

Pilocarpine, a topical drug, although systemically harmless and quite effective, causes considerable local difficulties. The pupil constricts so that little light becomes available to the eyes and the eye loses its ability to adapt from light to dark. Accommodation is stimulated so that the patient's refraction is sometimes incorrect and vision is blurred. The drug itself causes a local vasodilatation and red eyes and irritation are common. In short, although valuable, it really is unsatisfactory as a first line drug.

When carbonic anhydrase inhibitors are used systemically they have a number of disadvantages. While extremely effective in lowering intraocular pressure, they often cause a numbness and tingling, gastrointestinal upsets and, frequently, depression, lethargy, and a loss of appetite, and general malaise. These, in addition to the occasional more severe systemic complications such as aplastic anemia, are so common that many physicians are reluctant to routinely prescribe carboxy anhydrase inhibitors. As a consequence, only highly motivated patients who understand the seriousness of their condition will faithfully continue medication.

While investigators have long realized the benefits that would accompany topical administration, the selection of the proper entity has long eluded them. Most reports in the literature indicate that carbonic anhydrase inhibitors are inactive topically. Even the extreme measure of direct injection into the anterior chamber is reported to have no pressure lowering effect.

Silvestrini, "Effects of Topically Instilled Drugs on Intraocular Pressure in Rabbits", Arzeim.-Forsch. (Drug Res.) 25, Nr. 5 (1975), teaches that the disodium salts of acetazolamide can be used to lower the intraocular pressure of the normal rabbit eye. Silvestrini does, however, fail to confront the problem associated with the high pH of the disodium salt solutions and their high-likelihood of severe irritation and potential damage to the eye.

In the face of this collection of negative findings, it is now discovered that if monoalkali metal salt, most preferably the sodium or potassium salt, of a dibasic carbonic anhydrase inhibitor is employed as a topical agent, the pressure-lowering effects are substantially equivalent to systemic administration of the parent drug.

Because carbonic anhydrase inhibitors have a profound effect in altering basic metabolism, the avoidance of a systemic route serves to diminish, if not entirely eliminate, those side effects caused by metabolic acidosis such as vomiting, numbness, tingling, and general malaise and the like.

Furthermore, aqueous solutions of the dialkali metal salts are found to have a pH in excess of 10. Authors have asserted that if it is necessary to use a pH markedly at variance with the eye's normal pH of 7.4, medication is not well tolerated by the patient. Some consequences of applying medicaments with elevated pH's may be stinging, tissue irritation accompanied by loss of medication due to its being washed from the eye because of excessive tear production. While some alteration of the pH of a formulation of a dibasic salt of a carbonic anhydrase inhibitor can be achieved, it is recognized that this expedient has limits that affect the stability, solubility, and efficacy of the medicament.

In the practice of this invention, the alkali metal salts of carbonic anhydrase inhibitor are prepared from the known, salt-forming carbonic anhydrase inhibitors. As used herein, the term "alkali metal" includes lithium, sodium, potassium, cesium and rubidium, particularly sodium and potassium.

These salts may be hygroscopic and, as a consequence, may occur as hydrated species. However, when used in aqueous isotonic ophthalmic formulations, hygroscopicity is obviously no longer a problem. When formulating into an insert, the material should be kept as dry as possible during manufacture and protected from excessive moisture during storage and before use. The preferred alkali metal salts include as cations, potassium, sodium, and rubidium, with the most preferred being potassium and sodium.

Useful are those carbonic anhydrase inhibitors which have the sulfamoyl moiety (i.e., —SO$_2$NH$_2$) as a substituent. Thus, carbonic anhydrase inhibitors such as $p$-sulfamoylbenzoic acid and 5-benzene-sulfonamido-1,3,4-thiadiazol-2-sulfonamide can also be used when formulating the carbonic anhydrase inhibitor mono-alkali metal salts into an ophthalmically acceptable water soluble polymeric insert, e.g., from 0.1% to 5% by weight can be employed. The objective is to administer a dose of from 0.1 to 10 mg. per eye to the patient.

Thus, an ophthalmalogically acceptable insert according to the invention contains preferably from 0.1% to 15% of carbonic anhydrase inhibitor mono salt.

The compounds of this invention are administered in the form of ophthalmic pharmaceutical solid inserts. Formulations of these compounds may contain from 0.01 to 5% and especially 0.5% to 2% of medicament. Higher dosages as, for example, about 10%, or lower dosages can be employed provided the dose is effective in lowering intraocular pressure. As a unit dosage from between 0.001 to 10.0 mg., preferably .005 to 2.0 mg., and especially 0.1 to 1.0 mg. of the compound is generally applied to the human eye, generally on a daily basis.

These dosage values are believed accurate for human patients and are based on the pharmacology of carbonic anhydrase inhibitors and the action of other entities in the human eye. They reflect the best mode known. However, as with all medications dosage requirements are variable and must be individualized on the basis of the disease and the response of the patient. In rabbits the best mode is to employ a 5% to 10% by weight medicated insert, for reducing intraocular pressure in rabbits with $\alpha$-chymotrypsin induced ocular hypertension at a dose of 50 ml.

The pharmaceutical preparations are in the form of a solid insert. A solid water soluble polymer is used as the carrier for the medicament. Inserts that are known in the art that are suitable for use with this include those set forth and described in British Patent 15611, U.S.P. 3,993,071; U.S.P. 3,986,510; U.S.P. 3,868,445; and U.S.P. 3,867,510 employing the formulation and fabrication techniques described therein. The polymer used to form the insert may be any water soluble, ophthalmologically acceptable, non-toxic polymer, for example, cellulose derivatives such as methyl cellulose, alkali metal carboxymethyl cellulose, hydroxyethyl cellulose, hydroxypropyl cellulose, hydroxypropylmethyl cellulose; acrylates such as polyacrylic acid salts, ethylacrylates; polyacrylamides; natural products such as gelatin, alginates, pectins, tragacanth, karaya, chondrus, agar, acacia; the starch derivatives such as starch acetate, hydroxyethyl starch ethers, hydroxypropyl starch; as well as other synthetic derivatives such as polyvinyl alcohol, polyvinyl pyrrolidone, polyvinyl methyl ether, polyethylene oxide, neutralized carbopol and xanthan gum and mixtures of said polymer.

If a solid insert is employed, it preferably is prepared from cellulose derivatives such as methyl cellulose, hydroxyethyl cellulose and hydroxypropyl-cellulose.

Hydroxypropyl cellulose, one of the preferred polymers for the preparation of the insert is available in several polymeric forms, all of which are suitable in the preparation of these inserts. Thus, the product sold by Hercules, Inc. of Wilmington, Delaware, under the name KLUCEL® such as KLUCEL® HF, HWF, MF, GF, JF, LF and EF, which are intended for food or pharmaceutical use, are particularly useful. The molecular weight of these polymers useful for the purposes described herein may be at least 30,000 to about 1,000,000 or more.

Similarly, an ethylene oxide polymer having a molecular weight of up to 5,000,000 or greater, and preferably 100,000 to 5,000,000 can be employed.

Further, for example, POLYOX®, a polymer supplied by Union Carbide Co., may be used having a molecular weight of about 50,000 to 5,000,000 or more and preferably 3,000,000 to 4,000,000. Other specific polymers which are useful are polyvinyl pyrrolidine having a molecular weight of from about 10,000 to about 1,000,000 or more, preferably up to about 350,000 and especially about 20,000 to 60,000; polyvinyl alcohol having a molecular weight of from about 30,000 to 1,000,000 or more, particularly about 400,000 and especially from about 100,000 to about 200,000; hydroxypropylmethyl cellulose having a molecular weight of from about 10,000 to 1,000,000 or more, particularly up to about 200,000 and especially about 80,000 to about 125,000; methyl cellulose having a molecular weight of from about 10,000 to about 1,000,000 or more, preferably up to about 200,000 and especially about 50 to 100,000; and CARBOPOL® (carboxyvinyl polymer) of B. F. Goodrich and Co. designated as grades 934, 940 and 941.

For the purpose of this invention the type and molecular weight of the polymer is not critical. Any water soluble polymers can be used which have an average molecular weight which will afford dissolution of the polymer and, accordingly, the medicament in any desired length of time. The inserts, therefore, can be prepared to allow for retention and, accordingly, effectiveness in the eye for any desired period. The insert can be in the form of a square, rectangle, oval, circle, doughnut, semi-circle, 1/4 moon shape, and the like. Preferably, the insert is in the form of a rod, doughnut, oval or 1/4 moon. The insert can be readily prepared, for example, by dissolving the medicament and the polymer in a suitable solvent and evaporating the resulting solution to afford a thin film of the polymer which can then be subdivided to prepare inserts of appropriate size. Alternatively, the insert can be prepared by intimately admixing polymer and the medicament and thereafter molding the resulting mixture under the influence of heat and pressure to form a thin film. Preferably, the inserts are prepared by molding or extrusion procedures well known in the art. The molded or extruded product can then be subdivided to afford inserts of suitable size for administration in the eye.

The insert can be of any suitable size which readily fits into the eye. For example, castings or compression molded films having a thickness of about 0.25 mm. to 1.50 mm. can be subdivided to obtain suitable inserts. Rectangular segments of the cast or compressed film having a thickness between about 0.5 and 1.5 mm. can be cut to afford shapes such as rectangular plates of $4 \times 5$—20 mm. or ovals of comparable size. Similarly, extruded rods having a diameter between about 0.5 and 1.5 mm. can be cut into suitable sections to provide the desired amount of polymer. For example, rods of 1.0 to 1.5 mm. in diameter and about 2—20 mm. long are found to be satisfactory. The inserts may also be directly formed by injection molding. It is preferred that the ophthalmic inserts containing the medicament of the present invention be formed so that they are smooth and do not have any sharp edges or corners which could cause damage to the eye. Since the term smooth and sharp edges or corners are subjective terms, in this application these terms are used to indicate that excessive irritation of the eye will not result from the use of the insert.

The medicated ocular inserts can also contain plasticizers, buffering agents, appropriate inert fillers, and preservatives. Plasticizers suitable for this purpose must, of course, also be completely soluble in the lacrimal fluids of the eye. Examples of suitable plasticizers that might be mentioned are water, polyethylene glycol, propylene glycol, glycerine, trimethylol propane, di- and tripropylene glycol, hydroxypropyl sucrose and the like. Typically, such plasticizers can be present in the medicated ophthalmic insert in an amount ranging from up to 1 to about 30% by weight. A particularly preferred plasticizer is water which is present in amounts of at least about 5% up to about 40%. In actual practice, a water content of from about 10% to about 20% is preferred since it may be easily accomplished and adds the desired softness and pliability to the insert.

When plasticizing the solid medicinal product with water, the product is contacted with air having a relative humidity of at least 40% until said product picks up at least about 5% water and becomes softer and more pliable. In a preferred embodiment, the relative humidity of the air is from about 60% to about 99% and the contact is continued until the water is present in the product in amounts of from about 10% to about 20%.

Suitable water soluble preservatives which may be employed in the insert are alkali bisulfate, alkali thiosulfate, ascorbate, benzalkonium chloride, chlorobutanol, thimerosal, phenylmercuric acetate, phenylmercuric borate, parabens, benzyl alcohol and phenyl ethanol. These agents may be present in amounts of from 0.001 to 5% by weight of solid insert, and preferably 0.1 to 2%.

It is highly preferred that the solid inserts of this invention are available for use by the patient in a pathogen free condition. Thus, it is preferred to sterilize the inserts and so as insure against recontamination, the sterilization is preferably conducted after packaging. The best mode of sterilizing is to employ ionizing irradiation including irradiation emanating from cobalt 60 or high energy electron beams.

After packaging a convenient quantity of inserts, usually a single dose, the package is exposed to a sterilizing quantity of radiation. The preferred packaging employs sealing the inserts between layers of film or foil and then sealing or laminating the layers together about the edges. The techniques for performing the sterilization are well known and accepted, for example, as outlined in International Atomic Energy Commission, *Code of Practice for Radiosterilization of Medical Products*, 1967, pp. 423—431; and Block. *Disinfection, Sterilization and Preservation*, 2nd E., Lea & Febiger, Philadelphia, 1977, pp. 542—561.

The required quantity of irradiation can be determined experimentally by testing irradiated inserts for viable bacteria. Generally, the amount of irradiation desired to achieve sterilization is defined by the $D_{10}$ value. The $D_{10}$ value is the radiation dose that will reduce a given population of organisms by a factor of 10. Based on $D_{10}$ values, experimentally obtained for *Bacillus pumilus*, and presterilization contamination levels, a dose of 1.36 megarads is effective in obtaining a sterile product.

The following Examples illustrate the preparation of the mono-alkali metal salts of the carbonic anhydrase inhibitors of this invention.

Example 1
Preparation of 4,5-dichloro-m-benzenedisulfonamide mono sodium salt
To a solution of sodium methoxide (5.0 millimoles) in methanol (100 ml), freshly generated by the careful portionwise additions of sodium (1.15 g, 5.0 millimole) to methanol (100 ml.), is added 4,5-dichloro-m-benzenedisulfonamide (15.16 g, 5.0 millimole) with stirring at 25°C. The resulting mixture is stirred at 25°C for 30 minutes to provide a colorless solution which is filtered. Evaporation of the clear, colorless filtrate at 40°C *in vacuo* provides a solid residue which is dried *in vacuo* at 80°C for 16 hours to provide analytically pure 4,5-dichloro-m-benzenedisulfonamide mono sodium salt as a colorless solid (14.0 g., 85%), m.p./252—254°C.

Anal. Calcd. for $C_2H_5Cl_2N_2O_4S_2Na$: C, 22.03; H, 1.54; N, 8.56.

Found: C, 22.31; H, 1.55; N, 8.54.

Example 2

Preparation of 4,5-dichloro-m-benzenedisulfonamide mono potassium salt

Using essentially the procedure as described in Example 1, but replacing the sodium methoxide with an equivalent amount of potassium hydroxide, there is obtained 4,5-dichloro-m-benzenedisulfonamide mono potassium salt as a solid.

Example 3

Preparation of 4,5-dichloro-m-benzenedisulfonamide mono rubidium salt

Using essentially the procedure described in Example 1, but replacing the sodium methoxide with an equivalent amount of rubidium hydroxide, there is obtained 4,5-dichloro-m-benzenedisulfonamide mono rubidium salt.

In an analagous manner using equivalent quantities of materials, but substituting in place of the 4,5-dichloro-m-benzenedisulfonamide of Examples 1, 2 and 3, the following:

N-[5-(aminosulfonyl)-3-methyl-1,3,4-thiadiazol-2(3H)-ylidene]acetamide;
p-sulfamoylbenzoic acid
N-[5-(aminosulfonyl)-1,3,4-thiadiazol-2-yl]-propanamide;
N-[5-(aminosulfonyl)-1,3,4-thiadiazol-2-yl]-butanamide; and
5-benzenesulfonamido-1,3,4-thiadiazol-2-sulfonamide,

there are respectively prepared the monosodium, mono potassium and mono rubidium salts thereof.

In the reduction of intraocular pressure, and especially the treatment of mammalian glaucoma, additional agents particularly agents that work by a mode of action other than carbonic anhydrase inhibition can be employed in combination with the monosodium salts of this invention. In the practice of such combination therapy each agent will generally be employed in from 50 to 100% of the dosage that would have been employed had the respective agent been used in single entity therapy. It is feasible, however, to employ from 50 to 150% of the usual dosage of each entity when such is used in the combination. Examples of such additional therapeutic entities that may be employed in combination with carbonic anhydrase inhibitors are pilocarpine, particularly pilocarpine hydrochloride and other pilocarpine salts typically used in ophthalmology; epinephrine; and the $\beta$-blocking agents known to reduce intraocular pressure, particularly timolol.

**Claims for the Contracting States: BE CH DE FR GB IT LI LU NL SE**

1. An ophthalmologically acceptable water soluble polymeric insert for lowering intraocular pressure, characterized in that it comprises a mixture of a water soluble ophthalmologically acceptable non-toxic polymer and an intraocular pressure lowering amount of a mono alkali metal salt of a dibasic carbonic anhydrase inhibitor selected from

4,5-dichloro-m-benzene-disulfonamide;
N-[5-(aminosulfonyl)-3-methyl-1,3,4-thiadiazol-2(3H)-ylidene]acetamide;
p-sulfamoylbenzoic acid;
N-[5-(aminosulfonyl)-1,3,4-thiadiazol-2-yl]propanamide;
N-[5-(aminosulfonyl)-1,3,4-thiadiazol-2-yl]butanamide and
5-benzenesulfonamido-1,3,4-thiadiazol-2-sulfonamide.

2. An ophthalmologically acceptable water soluble polymeric insert for lowering intraocular pressure according to claim 1, characterized in that said mono alkali metal salt of the dibasic carbonic anhydrase inhibitor is from 0.1 to 15% by weight of the insert.

3. An ophthalmologically acceptable water soluble polymeric insert for lowering intraocular pressure according to one of claims 1 and 2, characterized in that said water soluble ophthalmologically acceptable non-toxic polymer is selected from cellulose derivatives such as methyl cellulose, alkali metal carboxymethyl cellulose, hydroxyethyl cellulose, hydroxypropyl cellulose, hydroxypropylmethyl cellulose; acrylates such as polyacrylic acid salts, ethylacrylates; polyacrylamides; natural products such as gelatin, alginates, pectins, tragacanth, karaya, chondrus, agar, acacia; the starch derivatives such as starch acetate, hydroxyethyl starch ethers, hydroxypropyl starch, as well as other synthetic

**0 036 351**

derivatives such as polyvinyl alcohol, polyvinyl pyrrolidone, polyvinyl methyl ether, polyethylene oxide, neutralized carbopol and xanthan gum and mixtures of said polymers.

4. An ophthalmologically acceptable water soluble polymeric insert for lowering intraocular pressure according to one of claims 1 to 3, characterized in that there is included an additional intraocular pressure lowering amount of an ophthalmic medicament that functions by a mechanism other than carbonic anhydrase inhibition.

5. An ophthalmologically acceptable water soluble polymeric insert for lowering intraocular pressure according to claim 4, characterized in that the additional ophthalmic medicament is timolol.

## Claims for the Contracting State: AT

1. A process for the preparation of an ophthalmologically acceptable water soluble polymeric insert for lowering intraocular pressure, characterized by mixing a water soluble ophthalmologically acceptable non-toxic polymer and an intraocular pressure lowering amount of a mono alkali metal salt of a dibasic carbonic anhydrase inhibitor selected from

4,5-dichloro-m-benzene-disulfonamide;
N-[5-(aminosulfonyl)-3-methyl-1,3,4-thiadiazol-2(3H)-ylidene]acetamide;
p-sulfamoylbenzoic acid;
N-[5-(aminosulfonyl)-1,3,4-thiadiazol-2-yl]propanamide;
N-[5-(aminosulfonyl)-1,3,4-thiadiazol-2-yl]butanamide and
5-benzenesulfonamido-1,3,4-thiadiazol-2-sulfonamide.

2. A process according to claim 1, characterized in that said mono alkali metal salt of the dibasic carbonic anhydrase inhibitor is from 0.1 to 15% by weight of the insert.

3. A process according to one of claims 1 and 2, characterized in that said water soluble ophthalmologically acceptable non-toxic polymer is selected from cellulose derivatives such as methyl cellulose, alkali metal carboxymethyl cellulose, hydroxyethyl cellulose, hydroxypropyl cellulose, hydroxypropylmethyl cellulose; acrylates such as polyacrylic acid salts, ethylacrylates; polyacrylamides; natural products such as gelatin, alginates, pectins, tragacanth, karaya, chondrus, agar, acacia; the starch derivatives such as starch acetate, hydroxyethyl starch ethers, hydroxypropyl starch, as well as other synthetic derivatives such as polyvinyl alcohol, polyvinyl pyrrolidone, polyvinyl methyl ether, polyethylene oxide, neutralized carbopol and xanthan gum and mixtures of said polymers.

4. A process according to one of claims 1 to 3, characterized in that there is included an additional intraocular pressure lowering amount of an ophthalmic medicament that functions by a mechanism other than carbonic anhydrase inhibition.

5. A process according to claim 4, characterized in that the additional ophthalmic medicament is timolol.

## Patentansprüche für die Vertragsstaaten: BE—CH—DE—FR—GB—IT—LI—LU—NL—SE

1. Ophthalmologisch brauchbare, wasserlösliche, polymere Einlage zur Senkung des intraokularen Drucks, dadurch gekennzeichnet, dass sie ein Gemisch aus einem wasserlöslichen, opthalmologisch brauchbaren, nicht-toxischen Polymeren und eine den intraokularen Druck senkende Menge eines Monoalkalimetallsalzes eines zweibasischen Carboanhydrase-Inhibitors, ausgewählt aus

4,5-Dichlor-m-benzol-disulfonamid;
N-[5-(Aminosulfonyl)-3-methyl-1,3,4-thiadiazol-2(3H)-yliden]acetamid;
p-Sulfamoylbenzoesäure;
N-[5-(Aminosulfonyl)-1,3,4-thiadiazol-2-yl]propanamid;
N-[5-(Aminosulfonyl)-1,3,4-thiadiazol-2-yl]butanamid und
5-Benzolsulfonamido-1,3,4-thiadiazol-2-sulfonamid, enthält.

2. Ophthalmologisch brauchbare, wasserlösliche, polymere Einlage zur Senkung des intraokularen Drucks gemäss Anspruch 1, dadurch gekennziechnet, dass das Monoalkalimetallsalz des zweibasischen Carboanhydrase-Inhibitors 0,1 bis 15 Gew.-% der Einlage beträgt.

3. Ophthalmologisch brauchbare, wasserlösliche, polymere Einlage zur Senkung des intraokularen Drucks gemäss einem der Ansprüche 1 und 2, dadurch gekennzeichnet, dass das wasserlösliche, ophthalmologisch brauchbare, nicht-toxische Polymere ausgewählt ist aus Cellulosederivaten, wie Methylcellulose, Alkalimetall-carboxymethylcellulose, Hydroxyäthylcellulose, Hydroxypropylcellulose, Hydroxypropylmethylcellulose; Acrylaten, wie Polyacrylsäuresalze, Äthylacrylate; Polyacrylamiden; natürlichen Produkten, wie Gelatine, Alginate, Pectine, Tragant, Karaya, Carragen, Agar, Gummiarabikum; den Stärkederivaten, wie Stärkeacetat, Hydroxyäthylstärkeäther, Hydroxypropylstärke sowie anderen synthetischen Derivaten, wie Polyvinylalkohol, Polyvinylpyrrolidon, Polyvinylmethyläther, Polyäthylenoxid, neutralisiertes Carbopol und Xanthangummi und Gemische dieser Polymeren.

6

4. Opthalmologisch brauchbare, wasserlösliche, polymere Einlage zur Senkung des intraokularen Drucks gemäss einem der Ansprüche 1 bis 3, dadurch gekennzeichnet, dass eine zusätzliche, den intraokularen Druck senkende Menge eines ophthalmischen Arzneimittels enthalten ist, das nach einem von der Carboanhydrase-Inhibierung unterschiedlichen Mechanismus wirkt.

5. Ophthalmologisch brauchbare, wasserlösliche, polymere Einlage zur Senkung des intraokularen Drucks gemäss Anspruch 4, dadurch gekennzeichnet, dass das zusätzliche ophthalmische Arzneimittel Timolol ist.

**Patentansprüche für den Vertragsstaat: AT**

1. Ein Verfahren zur Herstellung einer ophthalmologisch annehmbaren, wasserlöslichen, polymeren Einlage zur Erniedrigung des intraokularen Druckes, gekennzeichnet durch das Vermischen eines wasserlöslichen, ophthalmologisch annehmbaren, nichttoxischen Polymers und einer den intraokularen Druck erniedrigenden Menge eines Monoalkalimetallsalzes eines zweibasischen Carboanhydraseinhibitors, ausgewählt aus

4,5-Dichlor-m-benzoldisulfonamid;
N-[5-(Aminosulfonyl)-3-methyl-1,3,4-thiadiazol-2(3H)-yliden]acetamid;
p-Sulfamoylbenzoesäure;
N-[5-(Aminosulfonyl)-1,3,4-thiadiazol-2-yl]-propanamid;
N-[5-(Aminosulfonyl)-1,3,4-thiadiazol-2-yl]-butanamid und
5-Benzolsulfonamido-1,3,4-thiadiazol-2-sulfonamid.

2. Ein Verfahren nach Anspruch 1, dadurch gekennzeichnet, daß das genannte Monoalkalimetallsalz des zweibasischen Carboanhydraseinhibitors 0,1 bis 15 Gew.-% der Einlage ausmacht.

3. Ein Verfahren nach einem der Ansprüche 1 und 2, dadurch gekennzeichnet, daß das genannte wasserlösliche, ophthalmologisch annehmbare, nichttoxische Polymer aus Cellulosederivaten, wie Methylcellulose, Alkalimetallcarboxymethylcellulose, Hydroxyethylcellulose, Hydroxypropylcellulose, Hydroxypropylmethylcellulose; Acrylaten, wie Salze von Polyacrylsäure, Ethylacrylate; Polyacrylamiden; natürlichen Produkten, wie Gelatine, Alginate, Pektine, Tragant, Karaya, Chondrus, Agar, Akazia; den Stärkederivaten, wie Stärkeacetat, Hydroxyethylstärkeether, Hydroxypropylstärke; sowie anderen synthetischen Derivaten, wie Polyvinylalkohol, Polyvinylpyrrolidon, Polyvinylmethylether, Polyethylenoxid, neutralisiertes Carbopol und Xanthangummi; und Mischungen dieser Polymere; ausgewählt ist.

4. Ein Verfahren nach einem der Ansprüche 1 bis 3, dadurch gekennzeichnet, daß eine zusätzliche, den intraokularen Druck erniedrigende Menge eines ophthalmischen Arzneimittels einverleibt wird, das aufgrund eines von Carboanhydrasehemmung verschiedenen Mechanismus wirksam ist.

5. Ein Verfahren nach Anspruch 4, dadurch gekennzeichnet, daß das zusätzliche ophthalmische Arzneimittel Timolol ist.

**Revendications pour les Etats contractants: BE—CH—DE—FR—GB—IT—LI—LU—NL—SE**

1. Un insert polymère soluble dans l'eau acceptable en ophtalmologie pour abaisser la pression intraoculaire, caractérisé en ce qu'il comprend un mélange d'un polymère non toxique acceptable en ophtalmologie et soluble dans l'eau et d'une quantité abaissant la pression intra-oculaire d'un sel monométallique de métal alcalin d'un inhibiteur dibasique de l'anhydrase carbonique choisi parmi le

4,5-dichloro-m-benzène-disulfonamide;
le N-[5-(aminosulfonyl)-3-méthyl-1,3,4-thiadiazol-2(3H)-ylidène]acétamide;
l'acide p-sulfamoylbenzoïque;
le N-[5-(aminosulfonyl)-1,3,4-thiadiazol-2-yl]propanamide;
le N-[5-(aminosulfonyl)-1,3,4-thiadiazol-2-yl]butanamide et
le 5-benzènesulfonamido-1,3,4-thiadiazol-2-sulfonamide.

2. Un insert polymère soluble dans l'eau acceptable en ophtalmologie pour abaisser la pression intraoculaire selon la revendication 1, caractérisé en ce que ledit sel monométallique de métal alcalin de l'inhibiteur dibasique de l'anhydrase carbonique constitue 0,1 à 15% du poids de l'insert.

3. Un insert polymère soluble dans l'eau acceptable en ophtalmologie pour abaisser la pression intraoculaire selon l'une des revendications 1 et 2, caractérisé en ce que ledit polymère non toxique acceptable en ophtalmologie soluble dans l'eau est choisi parmi les dérivés de cellulose tels que la méthylcellulose, un dérivé de métal alcalin de la carboxyméthylcellulose, l'hydroxyéthylcellulose, l'hydroxypropylcellulose, l'hydroxypropylméthylcellulose; des acrylates tels que les sels de l'acide polyacrylique, les acrylates d'éthyle; les polyacrylamides; des produits naturels tels que la gélatine, les alginates, les pectines, la gomme adragante, la gomme de karaya, le chondrus, la gélose, la gomme arabique; les dérivés d'amidon tels que l'acétate d'amidon, les éthers hydroxyéthyliques de l'amidon,

**0 036 351**

l'hydroxypropylamidon, ainsi que d'autres dérivés synthétiques tels que l'alcool polyvinylique, la poly-vinylpyrrolidone, le poly (éther vinylméthylique), l'oxyde de polyéthylène, le Carbopol neutralisé et la gomme de xanthane et des mélanges desdits polymères.

4. Un insert polymère soluble dans l'eau acceptable en ophtalmologie pour abaisser la pression intraoculaire selon l'une des revendications 1 à 3, caractérisé en ce qu'il comprend une quantité additionnelle abaissant la pression intra-oculaire d'un médicament ophtalmique qui agit selon un mécanisme autre que l'inhibition de l'anhydrase carbonique.

5. Un insert polymère soluble dans l'eau acceptable en ophtalmologie pour abaisser la pression intraoculaire selon la revendication 4, caractérisé en ce que le médicament ophtalmique additionnel est le timolol.

**Revendications pour l'Etat contractant: AT**

1. Procédé pour la préparation d'un insert polymère soluble dans l'eau acceptable en ophtalmologie pour abaisser la pression intraoculaire, caractérisé en ce qu'on mélange un polymère non toxique acceptable en ophtalmologie et soluble dans l'eau et une quantité abaissant la pression intra-oculaire d'un sel monométallique de métal alcalin d'un inhibiteur dibasique de l'anhydrase carbonique choisi parmi le

4,5-dichloro-m-benzène-disulfonamide;
le N-[5-(aminosulfonyl)-3-méthyl-1,3,4-thiadiazol-2(3H)-ylidène]acétamide;
l'acide p-sulfamoylbenzoïque;
le N-[5-(aminosulfonyl)-1,3,4-thiadiazol-2-yl]propanamide;
le N-[5-(aminosulfonyl)-1,3,4-thiadiazol-2-yl]butanamide et
le 5-benzènesulfonamido-1,3,4-thiadiazol-2-sulfonamide.

2. Procédé selon la revendication 1, caractérisé en ce que ledit sel monométallique de métal alcalin de l'inhibiteur dibasique de l'anhydrase carbonique constitue 0,1 à 15% du poids de l'insert.

3. Procédé selon l'une des revendications 1 et 2, caractérisé en ce que ledit polymère non toxique acceptable en ophtalmologie soluble dans l'eau est choisi parmi les dérivés de cellulose tels que la méthylcellulose, un dérivé de métal alcalin de la carboxyméthylcellulose, l'hydroxyéthylcellulose, l'hydroxypropylcellulose, l'hydroxypropylméthylcellulose; des acrylates tels que les sels de l'acide polyacrylique, les acrylates d'éthyle; les polyacrylamides; des produits naturels tels que la gélatine, les alginates, les pectines, la gomme adragante, la gomme de karaya, le chondrus, la gélose, la gomme arabique; les dérivés d'amidon tels que l'acétate d'amidon, les éthers hydroxyéthyliques de l'amidon, l'hydroxypropylamidon, ainsi que d'autres dérivés synthétiques tels que l'alcool polyvinylique, la poly-vinylpyrrolidone, le poly (éther vinylméthylique), l'oxyde de polyéthylène, le Carbopol neutralisé et la gomme de xanthane et des mélanges desdits polymères.

4. Procédé pour la préparation d'un insert polymère selon l'une des revendications 1 à 3, caractérisé en ce qu'il comprend une quantité additionnelle abaissant la pression intra-oculaire d'un médicament ophtalmique qui agit selon un mécanisme autre que l'inhibition de l'anhydrase carbonique.

5. Procédé selon la revendication 4, caractérisé en ce que le médicament ophtalmique additionnel est le timolol.